**Europäisches Patentamt**

(19) **European Patent Office**

**Office européen des brevets**

(11) Veröffentlichungsnummer: **0 282 789 B1**

(12) **EUROPÄISCHE PATENTSCHRIFT**

(45) Veröffentlichungstag der Patentschrift:
04.07.90

(51) Int. Cl.⁵: **A61C 8/00**, A61B 17/58,
F16B 25/00

(21) Anmeldenummer: 88102858.3

(22) Anmeldetag: 26.02.88

(54) Selbstschneidendes einschraubbares Knochenimplantat für zahnärztliche Zwecke.

(30) Priorität: 17.03.87 DE 3708638

(43) Veröffentlichungstag der Anmeldung:
21.09.88 Patentblatt 88/38

(45) Bekanntmachung des Hinweises auf die Patenterteilung:
04.07.90 Patentblatt 90/27

(84) Benannte Vertragsstaaten:
AT CH DE FR GB IT LI SE

(56) Entgegenhaltungen:
EP-A- 0 104 552
EP-A- 0 111 134
CH-A- 321 629
DE-A- 3 136 602
DE-A- 3 531 389
DE-U- 8 523 007
GB-A- 602 614
US-A- 4 414 966
US-A- 4 537 185

(73) Patentinhaber: Grafelmann, Hans L., Prof.,
Parkstrasse 105, D-2800 Bremen 1(DE)

(72) Erfinder: Grafelmann, Hans L., Prof., Parkstrasse 105,
D-2800 Bremen 1(DE)

(74) Vertreter: Bauer, Rudolf, Dr. et al, Patentanwälte Dr.
Rudolf Bauer Dipl.-Ing. Helmut Hubbuch Dipl.-Phys.
Ulrich Twelmeier
Westliche-Karl-Friedrich-Strasse 29-31,
D-7530 Pforzheim(DE)

## Beschreibung

Die Erfindung bezieht sich auf ein einschraubbares Knochenimplantat für zahnärztliche Zwecke.

Bei der Pfostenverankerung zur Eingliederung zahnärztlicher Suprakonstruktionen wie z.B. Brücken oder Verstrebungen werden Zahnimplantate nach einer Tiefenbohrung in den Kieferknochen eingesetzt bzw. eingeschraubt. Ein einschraubbares Zahnimplantat besteht aus einem Pfostenlager mit einstückig angesetztem konischen Kern eines selbstschneidenden scharfgängigen Gewindes mit hoher Steigung. Das Pfostenlager hat ein Innengewinde, in das ein konischer Pfosten einschraubbar ist.

Bei den bekannten Schraubimplantaten dieser Art vgl. z.B. DE-A 3 136 602 verläuft das Gewinde mit seinem Kern konisch bzw. parallel zum Kern. Nur die äusseren Gewindegänge verbleiben über die ganze Länge der Tiefenbohrung in die Knochenwand selbst eingeschnitten. Es können dadurch freie Räume zwischen den Gewindegängen oder bleibende Kompressionen entstehen. In die Freiräume können sich von aussen Infektionen zwischen Knochenwand und Implantat einschieben.

Der Erfindung liegt die Aufgabe zugrunde, initial mehr Kontaktfläche zwischen Knochenwand und Implantat zu schaffen, den freien Ringraum zwischen Knochenwand und Implantat von Anfang an zu schließen und bleibende Kompressionen durch die Gewindegänge zu vermeiden.

Es soll eine breite gleichmäßige Knochenanlage an das Kieferimplantat sofort nach der Insertion erreicht werden. Diese ist besonders wichtig am Austrittspunkt aus dem Knochen und der Gingiva, also im oberen Teil der Insertion.

Diese Aufgaben sind bei dem Implantat der Erfindung dadurch gelöst, dass der Aussendurchmesser des Gewindes über die Länge des Kerns konstant gleich dem Durchmesser des zylindrischen Pfostenlagers ist. Diese Raumform wird am besten dadurch erreicht, dass sich die Gewindetiefe vom freien Ende des Kerns her bis zum zylindrischen Pfostenlager stetig bis auf Null vermindert.

Diese Aufgabe können erfindungsgemäß auch dadurch gelöst werden, daß der Aussendurchmesser des Gewindes, der am Ansatz gleich dem Durchmesser des zylindrischen Pfostenlagers ist, sich nach dem freien Ende des Kerns hin stetig vermindert.

Zur Erleichterung des Einschubvorganges kann sich der Implantatkörper nach dem freien Ende zu aber auch leicht verjüngen. Zu dem gleichen Zweck haben die Gänge des Gewindes, wie an sich bekannt (DE-A 3 136 602), gegeneinander versetzte Ausschnitte. Sie sind in weniger als 360° angeordnet, durchschnittlich sind also innerhalb von 360° zwei Ausschnitte vorgesehen.

Das Gewindeprofil ist dann nicht gleichschenklig vielmehr bildet die dem freien Ende des Kerns zugewandte Flanke der Gewindegänge einen Winkel von höchstens 90° die abgewandte Flanke einen stumpfen Winkel mit der Kernachse. Der stumpfe Winkel umfasst dabei etwa 120°.

Auch kann der Kern zu diesem Zweck einen oder mehrere Ausschnitte haben, der sich in den apikalbicortialen Abstützungsfortsatz erstreckt. Die Anschnittschärfe liegt dabei in der Richtung, in der das Implantat beim Einschrauben rotiert.

Das Pfostenlager hat eine sechseckige Ausnehmung zum Einsatz eines Schraubschlüssels beim Eindrehen des Implantats in die Knochenbohrung und an ihrem Boden ein Sackgewinde zum Einschrauben des temporären Heilungspfostens bzw. einer Deckschraube bzw. später des definitiven Pfostens. Diese Teile tragen das Aussengewinde an einem die sechseckige Ausnehmung durchsetzenden Stiel.

Das Implantat wird so weit eingeschraubt, daß die Ausnehmungsfläche mit der Knochenoberfläche bündig ist oder 1-2mm übersteht und die Spitze der Schraube möglichst die gegenüberliegende kompakte Knochenfläche berührt und sich daran abstützt. Bei Verwendung eines temporären Pfostens wird dessen Länge so gewählt, daß seine Oberfläche mit der Zahnfleischoberfläche bündig ist.

Statt eines Temporärpfostens, der aus dem Pfostenlager des Implantats bis zur Höhe der Gingivadicke herausragt, kann wahlweise eine flachere Deckschraube, wie an sich bekannt, mit einem Schraubenschlitz an ihrem oberen Ende verwendet werden, an dem zum Ein- und Ausdrehen ein Schraubenzieher eingreift. Die Deckschraube wird von der überwachsenden Gingiva vollständig bedeckt.

Beim Beschleifen von geschraubten Implantatpfosten mit rechtslaufenden Bohrinstrumenten kann es vorkommen, daß sich das Gewinde durch die Vibration lockert und angetrieben durch den rechtslaufenden Bohrer z.B. Luftturbine, in entgegengesetzter Richtung d.h. nach links, öffnet. Erfindungsgemäß kann dies dadurch verhindert werden, daß das Sackgewinde und das Außengewinde des Stiels linksgängig sind. In der Zeichnung ist:

Fig. 1 eine Ansicht des Implantats mit aufgebrochenem Pfostenlager und eingeschraubten Definitivpfosten mit Retentionsrillen und Schraubschlitz, mit im Durchmesser gleichlaufendem Pfostenübergang,

Fig. 2 eine perspektivische Ansicht des oberen Implantats mit Hexagonalausschnitt,

Fig. 3 eine Ansicht des teilweise geschnittenen Gewindes und

Fig. 4 ein gegenüber Fig. 3 vergrößerter Längsschnitt durch das Gewinde.

In der Zeichnung ist 2 ein am Anfang scharfgängiges Gewinde mit einem konischen Kern. Der Kern 3 ist einstückig mit dem zylindrischen Pfostenlager 4. Erfindungsgemäß nimmt die Gewindetiefe vom freien Ende des konischen Kerns 3 bis zum Zylinder 4 stetig bis auf Null ab, so daß der Aussendurchmesser des Implantatstabes über seine Länge konstant bleibt.

Die Gewindetiefe 0 wird 2-3mm unterhalb der Stoßstelle 14 erreicht.

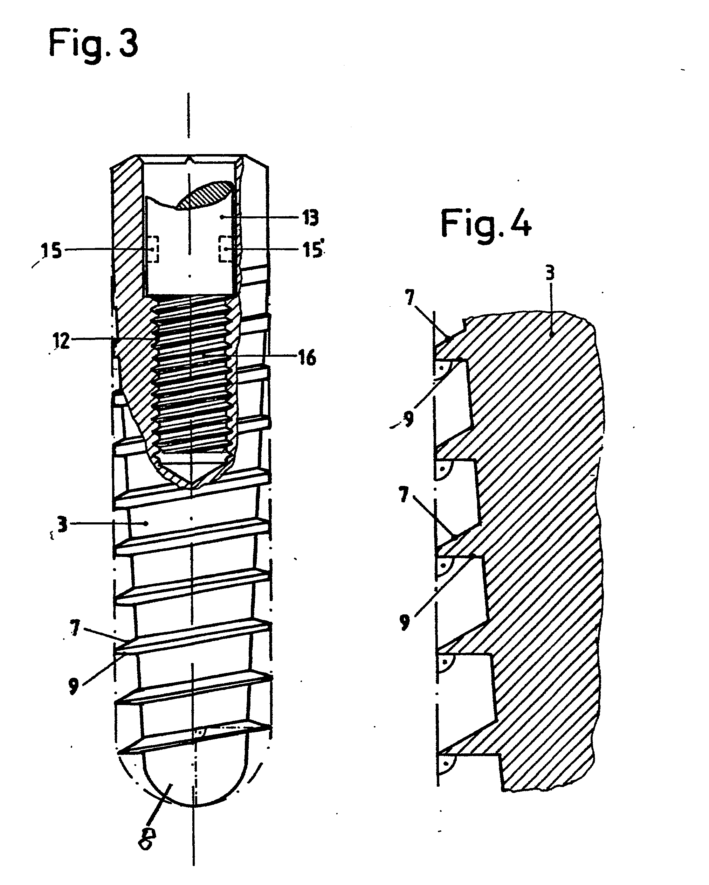

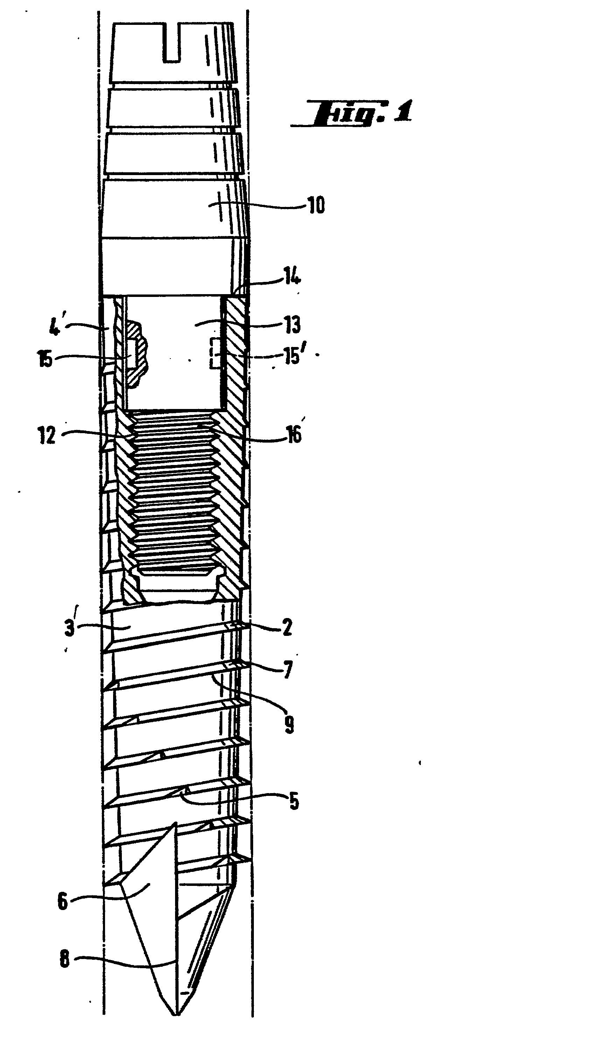

Zur Erleichterung des Selbstschneidens sind in den Gängen des Gewindes 2 keilartige Ausschnitte 5 vorgesehen. Zum gleichen Zweck hat der Kern 3 an seinem freien Ende Ausschnittkerben 6, deren Anschnittschärfe in der Rotationsrichtung des Implantats beim Einschrauben liegt. Die Gewindegänge 2 haben ein in Fig. 3 und 4 dargestelltes Profil. Die zum freien Ende des Kerns 3 hin gerichteten Flanken 9 schließen einen rechten Winkel mit der Kernachse ein, die zum Pfostenlager 4 hin gerichteten Flanken 7 einen stumpfen Winkel von 120°.

Der Pfosten 10 hat einen Stiel 13, der am freien Ende Aussengewinde 16 trägt. Das Pfostenlager 4 hat eine umrunde, hier sechseckige Ausnehmung 11 zum Einsatz eines Schraubwerkzeugs. Im Boden dieser Ausnehmung 11 befindet sich eine Sackbohrung mit Innengewinde 12. In dieses Gewinde (12) wird das Außengewinde 16 des Pfostens 10 eingeschraubt. Beiderseits der Stoßstelle 14 sind Kern 4 und Pfosten 10 Zylinder mit gleichem Durchmesser.

Im Stiel 13 des Pfostens 10 sind im Pfostenlager 4 Ausnehmungen 15, 15' zur Aufnahme des Klebezement vorgesehen, um eine spätere Rotation des Pfostens zu verhindern. Das Sacklochgewinde 12 und das Außengewinde 16 des Stiels 13 können aus den oben erwähnten Gründen Linksgewinde sein.

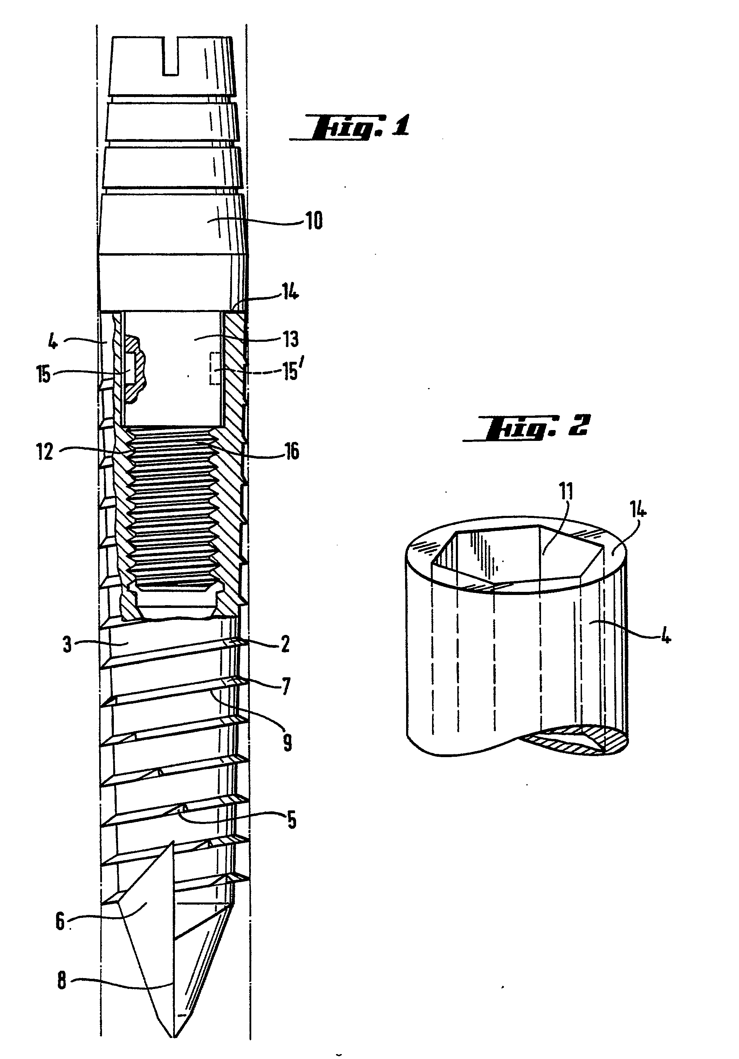

Das Freie Ende 8 des Kerns 3 ist entweder zugespitzt (Fig. 1) oder abgerundet (Fig. 3).

## Patentansprüche

1. Einschraubbares, selbstschneidendes Knochenimplantat zur Pfostenverankerung von zahnärztlichen Suprakonstruktionen, das aus einem zylindrischen Pfostenlager (4) mit einstückig angesetztem konischen Kern (3) eines selbstschneidenden scharfgängigen Gewindes (2) hoher Steigung besteht, dadurch gekennzeichnet, dass der Aussendurchmesser des Gewindes (2) über die Länge des Kerns (3) gleich dem Durchmesser des zylindrischen Pfostenlagers (4) ist.

2. Implantat nach Anspruch 1, dadurch gekennzeichnet, dass sich die Tiefe des Gewindes (2) vom freien Ende des Kerns (3) bis zum Pfostenlager (4) stetig bis auf Null vermindert.

3. Implantat nach Anspruch 1, dadurch gekennzeichnet, dass sich die Tiefe des Gewindes (2) vom freien Ende des Kerns (3) bis auf etwa 3 mm vor dem Pfostenlager stetig bis auf Null verringert.

4. Einschraubbares, selbstschneidendes Knochenimplantat zur Pfostenverankerung von zahnärztlichen Suprakonstruktion das aus einem zylindrischen Pfostenlager (4) mit einstückig angesetztem konischen Kern (3) eines selbstschneidenden scharfgängigen Gewindes (2) hoher Steigung besteht, dadurch gekennzeichnet, dass der Aussendurchmesser des Gewindes (2), der am Ansatz gleich dem Durchmesser des zylindrischen Pfostenlagers (4) ist, sich nach dem freien Ende des Kerns (3) hin stetig vermindert.

5. Implantat nach einem der vorhergehenden Ansprüche dadurch gekennzeichnet, daß zur Erleichterung der Selbstschneidung in den Gängen des Gewindes (2) gegeneinander versetzte Ausschnitte (5) vorgesehen sind.

6. Implantat nach einem der vorhergehenden Ansprüche dadurch gekennzeichnet, daß der Kern (3) an seinem freien Ende eine oder mehrere einlaufende Ausschnittkerben (6) hat.

7. Implantat nach Anspruch 5 dadurch gekennzeichnet, daß die dem freien Ende des Kerns (3) zugewandte Kante (9) der Gewinde(2)-gänge einen Winkel von 90° mit der Kernachse (8) einschließt, die abgewandte Flanke (7) einen stumpfen Winkel.

8. Implantat nach Anspruch 7 dadurch gekennzeichnet, daß der stumpfe Winkel, den die dem freien Kernende abgewandte Flanke (7) der Gewinde(2)-gänge einen Winkel von 90° mit der Kernachse (8) bildet, etwa 120° umfaßt.

9. Implantat nach einem der vorhergehenden Ansprüche, dadurch gekennzeichnet, daß sein Kern (3) eine nicht kreisförmig begrenzte axiale Ausnehmung (11) zum Einsatz eines Schraubwerkzeugs aufweist.

10. Implantat nach Anspruch 9, dadurch gekennzeichnet, daß am Boden der Ausnehmung (11) ein axiales Sackgewinde (12) angeordnet ist, in das (12) der mit einem Stiel (13) versehene Pfosten (10) eingeschraubt ist.

11. Implantat nach Anspruch 10, dadurch gekennzeichnet, daß Pfosten (10) und Kern (3) beiderseits der Stoßstelle (14) einachsige Zylinder gleichen Durchmessers sind.

12. Implantat nach Anspruch 10, dadurch gekennzeichnet, daß das Sacklochgewinde (12) und das Außengewinde (16) des Stiels (13) linksgängig sind.

13. Implantat nach Anspruch 10, dadurch gekennzeichnet, daß der im Kern (3) versenkte glatte Teil des Pfostens (10) radiale Ausnahmungen (15, 15) zur Aufnahme von Klebezement zwecks Rotationsverhinderung hat.

## Revendications

1. Implant osseux autotaraudeur vissable pour l'ancrage de piliers de supraconstructions dentaires, composé d'un support de pilier cylindrique (4) avec un noyau conique (3) d'un filet autotaraudeur pointu (2) à grande hauteur de pas, rapporté en une seule pièce, caractérisé en ce que, sur toute la longueur du noyau (3), le diamètre extérieur du filetage (2) est égal au diamètre du support de pilier cylindrique (4).

2. Implant selon la revendication 1, caractérisé en ce que la profondeur du filet (2) diminue progressivement jusqu'à zéro entre l'extrémité libre du noyau (3) et le support de pilier (4).

3. Implant selon la revendication 1, caractérisé en ce que la profondeur du filet (2) diminue progressivement jusqu'à zéro entre l'extrémité libre du noyau (3) et environ 3 mm avant le support de pilier.

4. Implant osseux autotaraudeur vissable pour l'ancrage de piliers de supraconstructions dentaires, composé d'un support de pilier cylindrique (4) avec un noyau conique (3) d'un filet autotaraudeur pointu (2) à grande hauteur de pas, rapporté en une seule pièce, caractérisé en ce que le diamètre extérieur du filet (2) qui, à l'embase, est égal au diamètre du support de pilier cylindrique (4), diminue progressivement en direction de l'extrémité du noyau (3).

5. Implant selon l'une des revendications précédentes, caractérisé en ce que, pour l'autotarau- dage dans les pas du filet (2), il est prévu des entailles (5) décalées les unes par rapport aux autres.

6. Implant selon l'une des revendications précédentes, caractérisé en ce que le noyau (3) présente à son extrémité libre une ou plusieurs encoches (6) d'approche.

7. Implant selon la revendication 5, caractérisé en ce que l'arête (9) des pas de vis (2) dirigée vers l'extrémité libre du noyau (3) forme avec l'axe (8) du noyau un angle de 90°, alors que le flanc opposé (7) forme avec ledit axe un angle obtus.

8. Implant selon la revendication 7, caractérisé en ce que l'angle obtus formé par le flanc (7) des pas de vis (2) opposé à l'extrémité libre du noyau et l'axe (8) du noyau, est d'environ 120°.

9. Implant selon l'une des revendications précédentes, caractérisé en ce que son noyau (3) présente un évidement (11) axial non délimité de manière circulaire pour l'introduction d'un instrument de vissage.

10. Implant selon la revendication 9, caractérisé en ce qu'au fond de l'évidement (11) est disposé un filet de trou borgne axial (12) dans lequel (12) est vissé le pilier (10) muni d'une queue (13).

11. Implant selon la revendication 10, caractérisé en ce que le pilier (10) et le noyau (3) constituent, de part et d'autre de la jonction (14), des cylindres avec un seul axe et avec des diamètres égaux.

12. Implant selon la revendication 10, caractérisé en ce que le filetage (12) du trou borgne et le filetage extérieur (16) de la queue (13) sont à pas à gauche.

13. Implant selon la revendication 10, caractérisé en ce que la partie lisse du pilier (10) noyée dans le noyau (3) présente des évidements radiaux (15, 15') destinés à recevoir du ciment adhésif pour empêcher un entraînement en rotation.

## Claims

1. A screw-in, self-cutting bone implant for post anchoring dental supraconstructions, comprising a cylindrical post bearing (4) having a conical core (3) of a self-cutting, angular, high-pitched thread, which is integrally attached thereto, characterized in that the outside diameter of the thread (2) across the length of the core (3) is equal to the diameter of the cylindrical post bearing (4).

2. Implant as claimed in Claim 1, characterized in that the depth of the thread (2) from the free end of the core (3) to the post bearing (4) continually reduces to zero.

3. Implant according to Claim 1, characterized in that the depth of the thread (2) from the free end of the core (3) to approximately 3 mm before the post bearing continually reduces to zero.

4. A screw-in, self-cutting bone implant for post anchoring dental supraconstructions, comprising a cylindrical post bearing (4) having a conical core (3) of a self cutting, angular, high-pitched thread (2), which is integrally attached thereto, characterized in that the external diameter of the thread (2), which is at the neck equal to the diameter of the cylindrical post bearing (4), continually reduces towards the free end of the core (3).

5. Implant as claimed in one of the preceding claims, characterized in that, for facilitating the self-cutting process, recesses (5) which are offset relative to each other, are provided in the threads (2).

6. Implant as claimed in one of the preceding claims, characterized in that the core (3) has at its free end one or several inwardly running notches (6).

7. Implant as claimed in Claim 5, characterized in that the edge (9) of the threads (2), which faces the free edge of the core (3), encompasses together with the core axis (8) an angle of 90°, the averse flank (7) an obtuse angle.

8. Implant as claimed in Claim 7, characterized in that the obtuse angle formed by the flank (7) of the threads (2), which faces away from the free core end, and the core axis (8) is approximately 120°.

9. Implant as claimed in one of the preceding claims, characterized in that its core (3) has an axial recess (11) for inserting a screw driver, said recess (11) being non circularly defined.

10. Implant as claimed in Claim 9, characterized in that an axial blind thread (12) is arranged at the bottom of the recess (11) into which the post (10) having a shank (13) is screwed.

11. Implant as claimed in Claim 10, characterized in that the post (10) and the core (3) are at both sides of the abutting face (14) single-axis cylinders of the same diameter.

12. Implant as claimed in Claim 10, characterized in that the thread (12) of the blind hole and the external thread (16) of the shank are left-hand threads.

13. Implant as claimed in Claim 10, characterized in that the smooth part of the post (10), which is sunk into the core (3), has radial recesses (15, 15) for receiving adhesive cement to prevent rotation.

Fig. 1

Fig. 2

# Fig. 3

# Fig. 4

*Fig. 1*